# EUROPEAN PATENT APPLICATION

(11) **EP 4 309 781 A1**
(43) Date of publication of application: **24.01.2024**
(21) Application number: 22771343.5
(22) Date of filing: 11.03.2022
(51) Int. Cl.: B01J 19/18, B01F 27/15, B01F 27/96, C12P 7/62

(54) **REACTION DEVICE AND METHOD FOR PRODUCING REACTION PRODUCT USING SAME**

(30) Priority: 17.03.2021 JP 2021044151; 17.06.2021 JP 2021101151; 26.01.2022 JP 2022010097
(71) Applicant: KANSAI CHEMICAL ENGINEERING CO. LTD, Amagasaki-shi Hyogo 660-0053 (JP); Bio-Energy Corporation, Amagasaki-shi, Hyogo 660-0053 (JP)
(72) Inventor: HAMA, Shinji, Amagasaki-shi, Hyogo 660-0053 (JP); MUKAIDA, Tadahiro, Amagasaki-shi, Hyogo 660-0053 (JP); NODA, Hideo, Amagasaki-shi, Hyogo 660-0053 (JP)
(74) Representative: Haywood, Carolyn
(86) International application number: PCT/JP2022/011147
(87) International publication number: WO 2022/196596

(57) **Abstract**

A reaction apparatus of the present invention includes a reaction chamber that contains a reaction liquid, and a liquid dispersion device provided inside the reaction chamber. The liquid dispersion device includes a rotary shaft disposed along a vertical direction, and at least one liquid flow member attached to the rotary shaft. The liquid flow member includes an ejection portion positioned above a liquid surface of the reaction liquid, a suction portion positioned below the liquid surface of the reaction liquid, and a flow path extending between the ejection portion and the suction portion and allowing the reaction liquid to flow therethrough.

## Description

### Technical Field

The present invention relates to a reaction apparatus and a method for producing a reaction product using the same.

### Background Art

Recently, fats and oils have been attracting attention as raw materials to be converted to fuels or chemicals. In particular, attempts to synthesize a long-chain fatty acid ester from an animal fat or oil and/or a vegetable fat or oil via a chemical reaction and use the synthesized long-chain fatty acid ester as a biodiesel fuel that can substitute for gas oil have been actively made.

Meanwhile, technologies for synthesizing various compounds through reactions such as asymmetric synthesis in reaction systems of two or more liquid phases using phase-transfer catalysts or slurry catalysts have also been attracting attention. In many of these reactions, the reaction is accelerated by vigorous stirring of the reaction system.

Two-liquid phase reactions may be employed in the production of biodiesel fuel, for example. Examples thereof include a transesterification reaction based on an enzyme-catalyzed process in which an enzyme such as lipase is employed as a catalyst. In such an enzyme-catalyzed transesterification reaction, for example, liquid enzymes or enzymes immobilized on supports such as ion-exchange resins (immobilized enzymes) are used as enzymes. A liquid enzyme is less expensive than an immobilized enzyme in that it is constituted by a concentrated and purified culture medium. In addition, the enzyme can be used in the next batch of reactions because it remains in the glycerol water that is a byproduct of the transesterification reaction described above. This allows repeated use of the liquid enzyme, thereby saving the cost of biodiesel fuel production (Non-Patent Document 1).

In the transesterification reaction using liquid enzymes, a two-phase system constituted by an oil layer and a water layer is used, and an emulsion is formed by stirring the reactants at high speed, for example. Such high-speed stirring of the reactants requires a considerable energy load on the stirrer. Meanwhile, to increase productivity as an industrial product, it is desired to reduce the energy required for operations such as stirring of the reactants. However, doing so reduces the emulsion-forming ability of the above reactants, resulting in the contradiction that the transesterification reaction cannot be effectively caused to occur.

Alternatively, an alkaline catalyst can be used in place of the above enzyme. In this case as well, a two-liquid phase reaction system is employed, and vigorous stirring is required for the reaction.

When slurry catalysts or immobilized enzymes with high specific gravity are used, they tend to settle to the bottom of the reactant (reaction liquid) without dissolving in the absence of stirring. Therefore, a large amount of energy is required to disperse them in the reaction system by stirring. Furthermore, there is a concern that vigorous stirring of immobilized enzymes with conventional stirring blades may result in undesired pulverization.

### Related Art Documents

### Non-Patent Documents

Non-Patent Document 1: M. Nordblad etal., Biotechnology and Bioengineering, 2014, Vol. 11, No. 12, pp.2446-2453

### Summary of Invention

### Problem to be Solved by the Invention

The present invention is to solve the above-descried problems, and it is an object thereof to provide a reaction apparatus that can reduce the energy required for operations such as mixing and stirring of reactants in the production of a reaction product, and a method for producing a reaction product using the reaction apparatus.

The present invention provides a reaction apparatus comprising: a reaction chamber that contains a reaction liquid; and a liquid dispersion device provided inside the reaction chamber,
wherein the liquid dispersion device includes a rotary shaft disposed along a vertical direction and at least one liquid flow member attached to the rotary shaft, and
the liquid flow member includes an ejection portion positioned above a liquid surface of the reaction liquid, a suction portion positioned below the liquid surface of the reaction liquid, and a flow path extending between the ejection portion and the suction portion and allowing the reaction liquid to flow therethrough.

In one embodiment, the liquid flow member is inclined such that the suction portion is closer to the rotary shaft than the ejection portion is.

In one embodiment, the liquid dispersion device includes a plurality of the liquid flow members around an axis of the rotary shaft.

In one embodiment, the liquid flow member has a cylindrical shape with open ends.

In one embodiment, the liquid flow member has a gutter-like shape.

In one embodiment, the liquid flow member is constituted by at least one first liquid flow member and at least one second liquid flow member, and the suction portion of the first liquid flow member is disposed above the suction portion of the second liquid flow member.

In one embodiment, the reaction liquid is constituted by a plurality of liquid phases.

The present invention also provides a liquid dispersion device comprising: a rotary shaft disposed along a vertical direction; and at least one first liquid flow member and at least one second liquid flow member that are attached to the rotary shaft,
wherein the first liquid flow member and the second liquid flow member each include an ejection portion positioned at an upper end thereof, a suction portion positioned at a lower end, and a flow path extending between the ejection portion and the suction portion, and
the suction portion of the first liquid flow member is disposed above the suction portion of the second liquid flow member.

In one embodiment, the first liquid flow member and the second liquid flow member are each inclined such that the suction portion is closer to an axis of the rotary shaft than the ejection portion is.

In one embodiment, the first liquid flow member and the second liquid flow member each have a cylindrical shape with open ends.

The present invention also provides a method for producing a reaction product, comprising stirring a reaction liquid through circulation inside the above reaction apparatus.

In one embodiment, the reaction liquid is constituted by a plurality of liquid phases.

In one embodiment, the reaction product is a fatty acid ester, and the reaction liquid contains a raw material fat or oil, a liquid enzyme, an alcohol having 1 to 8 carbon atoms, and water.

In a further embodiment, the raw material fat or oil is at least one fat or oil selected from the group consisting of vegetable fats and oils, animal fats and oils, fish oils, fats and oils produced by microorganisms, and waste oils thereof.

### Effects of the Invention

According to the present invention, the reaction liquid can be efficiently mixed up by moving the scooped up reaction liquid to a position above the liquid surface and dispersing it. For example, in addition to stirring based on horizontal rotation, vertical movement and circulation can be facilitated for the reaction liquid contained in the reaction chamber. This can reduce the energy required for the physical operations such as mixing and stirring of the reactants in the production of various reaction products. For example, when a reaction liquid constituted by a plurality of liquid phases (such as two phases of oil and water) is contained in the reaction apparatus of the present invention, emulsification of the reaction liquid can be facilitated without the use of extra power.

### Brief Description of Drawings

FIG. 1 is a schematic view showing an example of a reaction apparatus of the present invention.
FIG. 2(a) is an end view of the reaction apparatus taken along the line A-A in FIG. 1, and FIG. 2(b) is an end view of the reaction apparatus taken along the line B-B in FIG. 1.
FIG. 3 is a perspective view schematically showing an example of a liquid flow member constituting a liquid dispersion device of the reaction apparatus shown in FIG. 1.
FIG. 4 is a schematic view showing another example of the reaction apparatus of the present invention.
FIG. 5 is a schematic view of an inverted skirt that can be incorporated into the reaction apparatus of the present invention.
FIG. 6 is a schematic view showing an example of the reaction apparatus of the present invention incorporating the inverted skirt shown in FIG. 5.
FIG. 7 is a schematic view showing another example of the reaction apparatus of the present invention.
FIG. 8 is a schematic view showing another example of the reaction apparatus of the present invention.
FIG. 9(a) is an end view of the reaction apparatus taken along the line A-A in FIG. 8, and FIG. 9(b) is an end view of the reaction apparatus taken along the line B'-B' in FIG. 8.
FIG. 10 is a perspective view schematically showing an example of a liquid flow member constituting a liquid dispersion device of the reaction apparatus shown in FIG. 8.
FIG. 11 is a schematic view showing another example of the reaction apparatus of the present invention.
FIG. 12 is a schematic view showing another example of the reaction apparatus of the present invention.
FIG. 13 is a schematic view showing another example of the reaction apparatus of the present invention.
FIG. 14 is a schematic view showing an example of the reaction apparatus of the present invention incorporating another liquid dispersion device.
FIG. 15 shows schematic views of test apparatuss fabricated in Reference Examples 1 to 3, where FIG. 15(a) is a schematic view of a test apparatus (R1) fabricated in Reference Example 1, FIG. 15(b) is a schematic view of a test apparatus (R2) fabricated in Reference Example 2, FIG. 15(c) is a schematic view of a test apparatus (R3) fabricated in Reference Example 3, and FIG. 15(d) is an enlarged perspective view of an anchor-type (U-shaped) stirring blade used in Reference Example 3.
FIG. 16 is a graph showing a relationship between the number of rotations in methyl ester and the torque of the test apparatuss (R1) and (R3) fabricated in Reference Examples 1 and 3.
FIG. 17 is a graph showing a change in the amount of methyl ester produced in the transesterification reaction in Example 1 and Comparative Examples 1 and 2.
FIG. 18 is a graph showing a particle size distribution of a W/O emulsion of the reaction liquid obtained in Example 1.
FIG. 19 is a microphotograph of the W/O emulsion of the reaction liquid obtained in Example 1.
FIG. 20 shows photographs showing a state in which the rotation of a rotary shaft was stopped after the transesterification reaction in Example 2, where FIG. 20(a) is a photograph showing a state of the reaction liquid when 1 minute had elapsed after the rotation of the rotary shaft was stopped, FIG. 20(b) is a photograph showing a state of the reaction liquid when 5 minutes had elapsed after the rotation of the rotary shaft was stopped, and FIG. 20(c) is a photograph showing a state of the reaction liquid when 1 minute had elapsed after the rotation of the rotary shaft was stopped.

### Description of Embodiments

### (Reaction Apparatus)

Hereinafter, a reaction apparatus of the present invention will be described with reference to the accompanying drawings. Note that constituent elements denoted by the same reference numerals in the following drawings are the same as those shown in the other drawings.

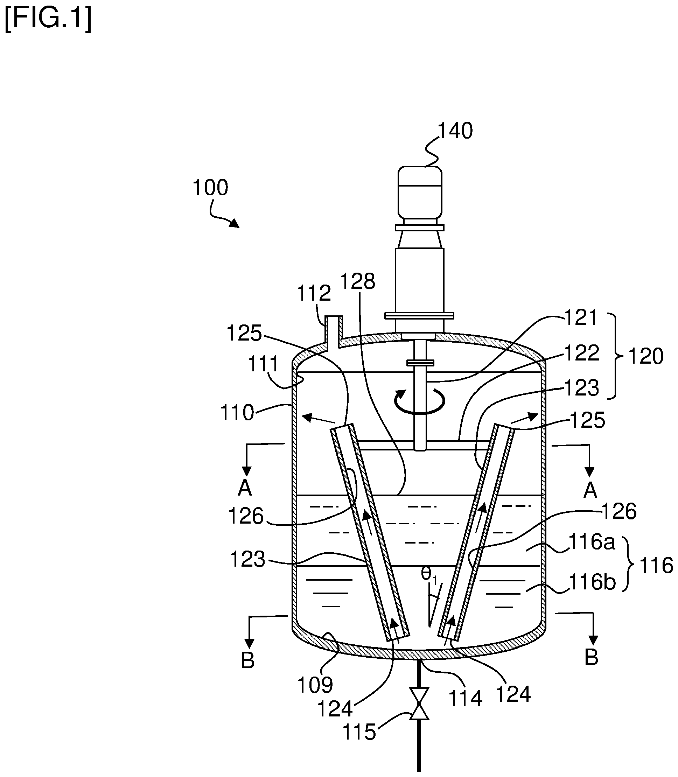

FIG. 1 is a schematic view showing an example of a reaction apparatus of the present invention. A reaction apparatus 100 includes a reaction chamber 110 and a liquid dispersion device 120.

The reaction chamber 110 is a sealable chamber in which a reaction liquid 116 can be contained and stirred, and has a bottom 109 that is a flat bottom, a round bottom, a conical bottom, or a downward sloping bottom, for example.

The size (capacity) of the reaction chamber 110 is not necessarily limited, because it is set as appropriate according to the application of the reaction apparatus 100 (e.g., the type of reaction to be caused to occur using the reaction apparatus), the amount of reaction liquid processed, and the like, but the size is from 0.1 liters to 40,000 liters, for example.

In an embodiment, the reaction chamber 110 further includes a reaction liquid inlet 112 and a product outlet 114. The reaction liquid inlet 112 is an inlet through which the reaction liquid 116 is to be newly supplied into the reaction chamber 110. The reaction liquid inlet 112 is provided on the top (e.g., on the top lid) of the reaction chamber 110, for example. Alternatively, the reaction liquid inlet 112 may be provided on a side face of the reaction chamber 110. The number of reaction liquid inlets 112 provided on the reaction chamber 110 is not limited to one. For example, a plurality of reaction liquid inlets may be provided on the reaction chamber 110.

The product outlet 114 is an outlet through which a product obtained in the reaction chamber 110 is to be taken out of the reaction chamber 110. The product outlet 114 is capable of discharging reaction residues, effluent, and the like in addition to the product, and the discharge can be regulated by opening and closing a valve 115 provided downstream of the product outlet 114, for example. The product outlet 114 is provided in connection with the center of the bottom 109 in the reaction chamber 110, for example.

The top of the reaction chamber 110 may have an openable structure, such as a lid or a maintenance hole, for example. The top of the reaction chamber 110 may further have a pressure adjustment port (not shown) for adjusting the pressure in the reaction chamber 110. Furthermore, the pressure adjustment port may be connected to an unshown pressure reducing pump, for example.

The liquid dispersion device 120 is provided inside the reaction chamber 110, and is constituted by a rotary shaft 121 disposed along the vertical direction inside the reaction chamber 110, and liquid flow members 123 attached to the rotary shaft 121 via an attachment 122 extending preferably in the horizontal direction. The rotation of the rotary shaft 121 and centrifugal force caused by the rotation and applied to the liquid flow members 123 can cause the liquid dispersion device 120 to scoop up the reaction liquid 116 contained in the reaction chamber 110 and cause the liquid to flow upward from the lower side of the reaction chamber 110. The liquid flow members 123 each include a suction portion 124, an ejection portion 125, and a flow path 126 extending between the suction portion 124 and the ejection portion 125. The suction portion 124 is disposed below a liquid surface 128 of the reaction liquid 116, and the ejection portion 125 is disposed above the liquid surface 128 of the reaction liquid 116.

In the present invention, the arrangement of the suction portion 124 and the ejection portion 125 described above is preferably maintained not only in a static stage (i.e., a state in which the rotary shaft 121 is not rotated and the liquid surface of the reaction liquid 116 is spread substantially in the horizontal direction) but also in a stage in which the rotary shaft 121 is rotated at a desired rotational speed (i.e., a state in which the reaction liquid 116 is stirred as described later through the rotation of the rotary shaft 121). As a result, the reaction liquid 116 in the reaction chamber 110 can be easily scooped up from the suction portions 124 of the liquid flow members due to the rotation of the rotary shaft 121 and the liquid flow members 123, moved by centrifugal force through the flow paths 126 in the liquid flow members 123 to the ejection portions 125, and then ejected from the ejection portions 125 toward an inner wall 111 of the reaction chamber 110 and the liquid surface 128 of the reaction liquid 116 (an oil phase 116a), for example.

The rotary shaft 121 is a shaft with a predetermined rigidity, and has, for example, a circular cylindrical or solid cylindrical shape. The rotary shaft 121 is typically disposed along the vertical direction inside the reaction chamber 110. The thickness of the rotary shaft 121 is not necessarily limited, but is 8 to 200 mm, for example. The length of the rotary shaft 121 varies in accordance with the size of the reaction chamber 110 used or the like, and an appropriate length may be selected by a person skilled in the art.

In the reaction apparatus 100 of the present invention, an end of the rotary shaft 121 is connected to a motor 140 or other rotating means at a position above the reaction chamber 110. The other end of the rotary shaft 121 is not connected to the bottom 109 of the reaction chamber 110, and is disposed at a certain distance from the bottom 109 of the reaction chamber 110 (preferably disposed at a distance from the liquid surface 128 of the reaction liquid 116), for example. This reduces the risk of the rotary shaft 121 coming into contact with the reaction liquid 116. Alternatively, the other end of the rotary shaft may be housed in a bearing provided at the bottom 109 of the reaction chamber.

In the reaction apparatus 100 shown in FIG. 1, the liquid flow members 123 are each inclined such that the suction portion 124 is closer to the rotary shaft 121 than the ejection portion 125 is. In FIG. 1, the liquid flow members 123 are each attached so as to be inclined to form a predetermined angle (alternatively referred to as an "attachment inclination angle") θ₁ with respect to the axial direction of the rotary shaft 121. The attachment inclination angle θ₁ may be set to any angle by a person skilled in the art, but is, for example, 10 to 45° and preferably 15 to 25°.

In the reaction apparatus 100 shown in FIG. 1, as the liquid dispersion device 120, two liquid flow members 123 are provided symmetrically around the rotary shaft 121. The number of liquid flow members that can be provided in the reaction apparatus of the present invention is not necessarily limited, but is, for example, one or more, preferably from 2 to 8, and more preferably from 2 to 6. These liquid flow members are preferably attached at substantially equal angle intervals around the axis of the rotary shaft.

In the present invention, the liquid flow members 123 may be processed to be entirely cylindrical (e.g., circular cylindrical, elliptic cylindrical, or angular cylindrical), may have a so-called gutter-like shape such as a semi-circular cylindrical, semi-angular cylindrical, or V letter shape, may have such a gutter-like shape at the lower end and the upper end with an intermediate portion therebetween being processed to be cylindrical (e.g., circular cylindrical, elliptic cylindrical, or angular cylindrical), or may have such a gutter-like shape only at the lower end or only at the upper end with the other portion being processed to be cylindrical (e.g., circular cylindrical, elliptic cylindrical, or angular cylindrical), for example.

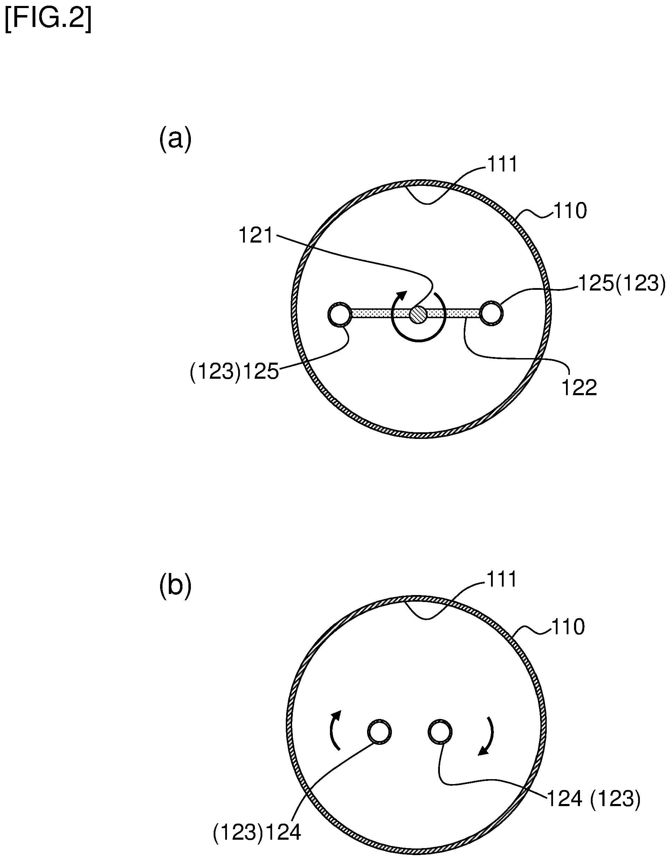

For example, in the embodiment shown in FIG. 1, the liquid flow members 123 have a cylindrical shape with open ends. If such cylindrical liquid flow members 123 are employed, two liquid flow members 123 are attached around the axis of the rotary shaft 121 by means of the attachment 122 at a substantially equal distance from the rotary shaft 121 in a horizontal end face in the upper portion of the reaction chamber 110 in the reaction apparatus 100 (e.g., near the ejection portions 125 of the liquid flow members 123 taken along the line A-A in FIG. 1) as shown in FIG. 2(a), for example. When the rotary shaft 121 rotates, the two liquid flow members 123 can be rotated via the attachment 122 along a portion closer to the inner wall 111 than to the center of the reaction chamber 110. Meanwhile, the two liquid flow members 123 are positioned close to the center of the reaction chamber 110 in a horizontal end face in the lower portion of the reaction chamber 110 in the reaction apparatus 100 (e.g., near the suction portions 124 of the liquid flow members 123 taken along the line B-B in FIG. 1) as shown in FIG. 2(b), for example. When the rotary shaft 121 rotates, the two liquid flow members 123 can be rotated along a portion close to the center of the reaction chamber 110.

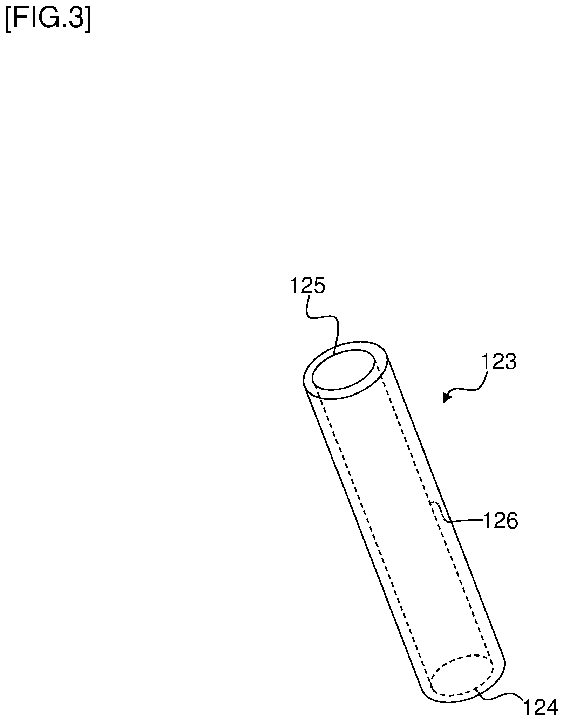

The size of the liquid flow members 123 is not particularly limited, but, for example, if a circular cylindrical member as shown in FIG. 3 is employed, the inner diameter of the circular cylindrical portion is 2 to 200 mm, for example. The length from the suction portion 124 to the ejection portion 125 (i.e., the length of the path 126) is 40 to 8,000 mm, for example.

Referring again to FIG. 1, the reaction liquid 116 contained in the reaction chamber 110 is liquid such as an aqueous solution or slurry. If the reaction apparatus 100 of the present invention is used for the production of fatty acid esters as described below, for example, the reaction liquid 116 is constituted by a two-phase system including an oil phase 116a and a water phase 116b, for example, and both of the oil phase 116a and the water phase 116b contain reactants such as starting materials and media such as solvents. The reaction liquid 116 contained in the reaction chamber 110 is not limited to such a two-phase system including the oil phase 116a and the water phase 116b. The reaction liquid 116 may be constituted by one liquid phase or a plurality of liquid phases (e.g., two or three phases).

According to the reaction apparatus 100 of the present invention, the rotation of the rotary shaft 121 causes the liquid flow members 123 of the liquid dispersion device 120 to scoop up the reaction liquid 116 from the suction ports 124. The scooped up reaction liquid is moved through the paths 126 to the ejection ports 125 by centrifugal force caused by the rotation of the rotary shaft 121, and then ejected from the ejection ports 125 into the reaction chamber 110, specifically, to portions above the liquid surface 128 of the reaction liquid 116 in the reaction chamber 110. This allows the reaction liquid 116 to collide with the inner wall 111 of the reaction chamber 110 and the liquid surface 128 and to move upward from the bottom 109 of the reaction chamber 110, thereby facilitating mixing up (e.g., stirring or circulation in the vertical direction) of the reaction liquid 116 in the height direction of the reaction chamber 110. As a result, the production of reaction products performed in the reaction apparatus 100 can be progressed more effectively.

The reaction chamber 110, the rotary shaft 121, the attachment 122, and the liquid flow members 123 are each independently made of materials such as iron, stainless steel, hastelloy, titanium, or other metals, or combinations thereof, for example. These components may be given coatings known in the art, such as Teflon (registered trademark), glass lining, or rubber lining, to enhance chemical resistance.

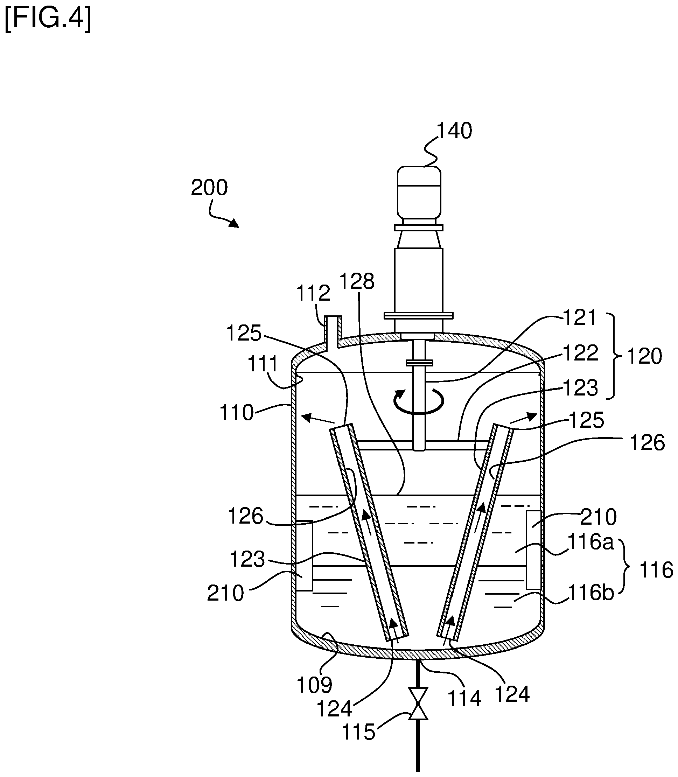

FIG. 4 is a schematic view showing another example of the reaction apparatus of the present invention. In a reaction apparatus 200 of the present invention shown in FIG. 4, a plurality of obstruction plates 210 are provided on the inner wall 111 of the reaction chamber 110. In FIG. 4, the obstruction plates 210 are positioned at substantially the center of the reaction liquid 116 that is allowed to stand (i.e., such that the upper ends of the obstruction plates 210 are positioned in the oil phase 116a, the lower ends of the obstruction plates 210 are positioned in the water phase 116b, and the upper ends and the lower ends are positioned close to the interface between the oil phase 116a and the water phase 116b), for example.

When the rotary shaft 121 rotates the liquid flow members 123 via the attachment 122, the obstruction plates 210 play a role of preventing the reaction liquid 116 in the reaction chamber 110 from rotating following the liquid flow members. In other words, the obstruction plates 210 serve as obstacles when the reaction liquid 116 rotates in the horizontal direction inside the reaction chamber 110, preventing the formation of vortices. As a result, the reaction liquid 116 is caused to irregularly move inside the reaction chamber 110, resulting in increased stirring efficiency of the reaction liquid 116.

The number of obstruction plates 210 provided in the reaction chamber 110 is not necessarily limited, but is 1 to 8 at substantially equal intervals on the inner wall 111 of the reaction chamber 110, for example.

In the present invention, the following components may be placed outside the liquid dispersion member instead of or together with the use of the above-described obstruction plates.

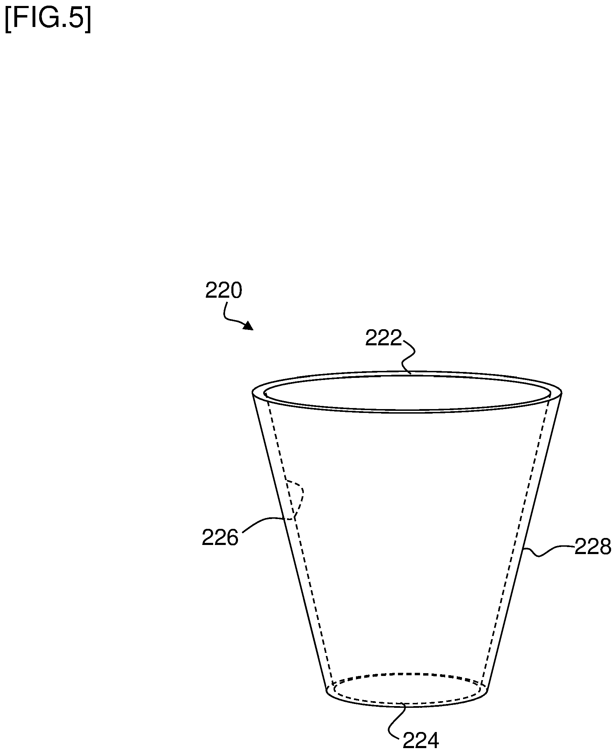

FIG. 5 is a schematic view of an inverted skirt that can be incorporated into the reaction apparatus of the present invention.

An inverted skirt 220 shown in FIG. 5 has a hollow inverted truncated cone shape, and is constituted by an upper opening 222 located on the upper side, a lower opening 224 located on the lower side, and an inner inclined face 226 and an outer inclined face 228 located between the upper opening 222 and the lower opening 224.

Both the upper opening 222 and the lower opening 224 preferably have a circular or oval shape, and more preferably have a circular shape. The inverted skirt 220 is designed to expand in diameter from the lower opening 224 to the upper opening 222.

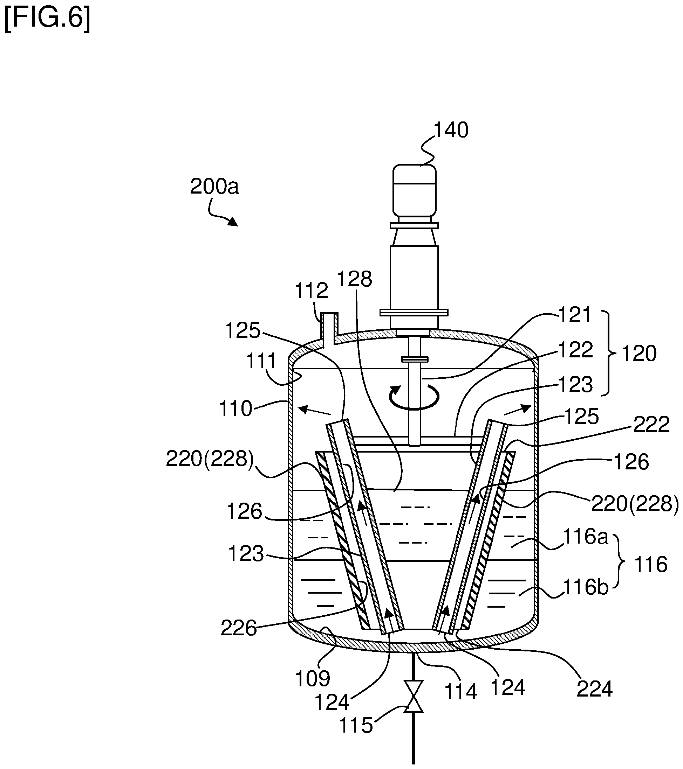

FIG. 6 is a schematic view showing an example of the reaction apparatus of the present invention incorporating the inverted skirt shown in FIG. 5.

In a reaction apparatus 200a shown in FIG. 6, the inverted skirt 220 is disposed outside the liquid dispersion members 123 in the liquid dispersion device 120, and fixed by an unshown fixing means to the liquid dispersion members 123 and/or the attachment 122, for example.

The suction portions 124 of the liquid dispersion members 123 are arranged at substantially the same height as the lower opening 224 of the inverted skirt 220, arranged projecting from the lower opening 224 of the inverted skirt 220, or arranged so as to be positioned above the lower opening 224 of the inverted skirt 220.

Meanwhile, the ejection portions 125 of the liquid dispersion members 123 are arranged projecting from the upper opening 222 so as to be positioned above the upper opening 222 of the inverted skirt 220. In FIG. 6, there is space between the liquid dispersion members 123 and the inner inclined face 226 of the inverted skirt 220, but the present invention is not limited to this. The liquid dispersion members 123 and the inner inclined face 226 of the inverted skirt 220 may be in close contact with each other without any space therebetween.

In the embodiment shown in FIG. 6, due to the rotation of the rotary shaft 121, the liquid dispersion device 120 rotates, and the inverted skirt 220 rotates as well inside the reaction chamber 110. At that time, the outer inclined face 228 of the inverted skirt 220 can rotate relatively quietly inside the reaction liquid 116. This can suppress or reduce the formation of vortices by the reaction liquid 116 in the reaction chamber 110 without providing the obstruction plates 210 shown in FIG. 4 in the reaction chamber 110, resulting in increased stirring efficiency of the reaction liquid 116.

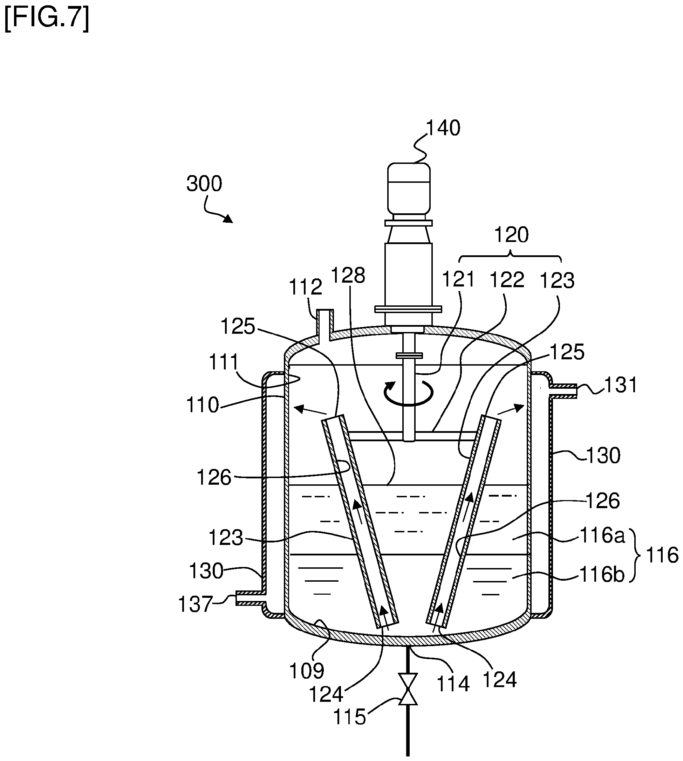

FIG. 7 is a schematic view showing another example of the reaction apparatus of the present invention. In the embodiment shown in FIG. 7, a jacket 130 for temperature control is provided around the outer circumference of the reaction chamber 110. In FIG. 7, the jacket 130 is made of a hollow material, for example, and is configured such that a thermal medium such as steam, water, or heat transfer oil can be introduced via an unshown pipe from a jacket inlet 131 and discharged from a jacket outlet 137, for example. The thermal medium introduced into the jacket 130 can control the temperature of the reaction liquid 116 in the reaction chamber 110, by heating the reaction liquid 116 from outside of the reaction chamber 110.

In the embodiment shown in FIG. 7, an example was described in which a thermal medium for heating is used as a thermal medium to heat the internal portion of the reaction chamber 110, but the present invention is not limited to this. Instead of the thermal medium for heating, a thermal medium for cooling, such as water, brine, or gas refrigerant (e.g., carbon dioxide, chlorofluorocarbon) may be introduced into the jacket 130.

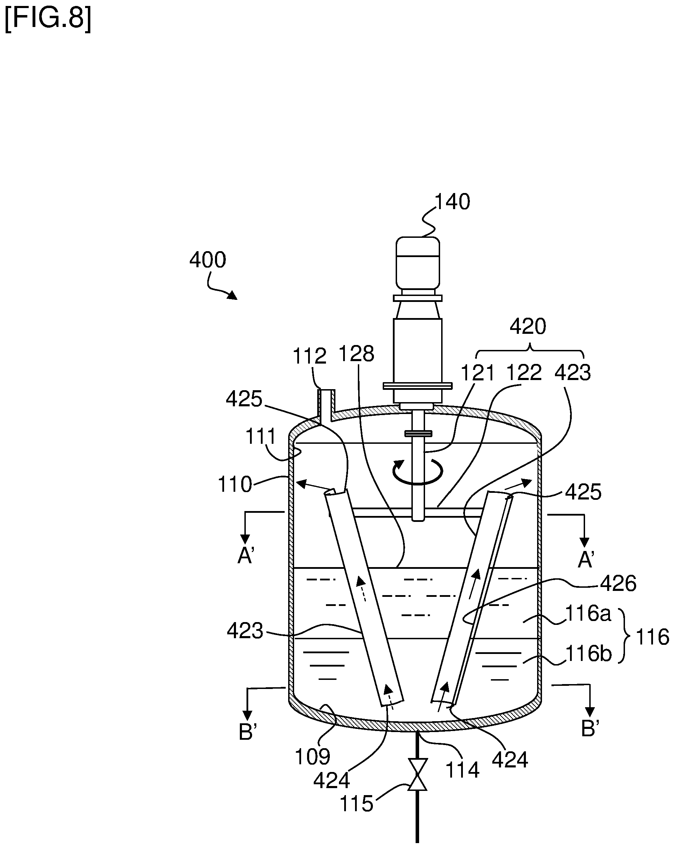

FIG. 8 is a schematic view showing another example of the reaction apparatus of the present invention. In a reaction apparatus 400, flow paths 426 of liquid flow members 423 constituting a liquid dispersion device 420 each have a gutter-like shape.

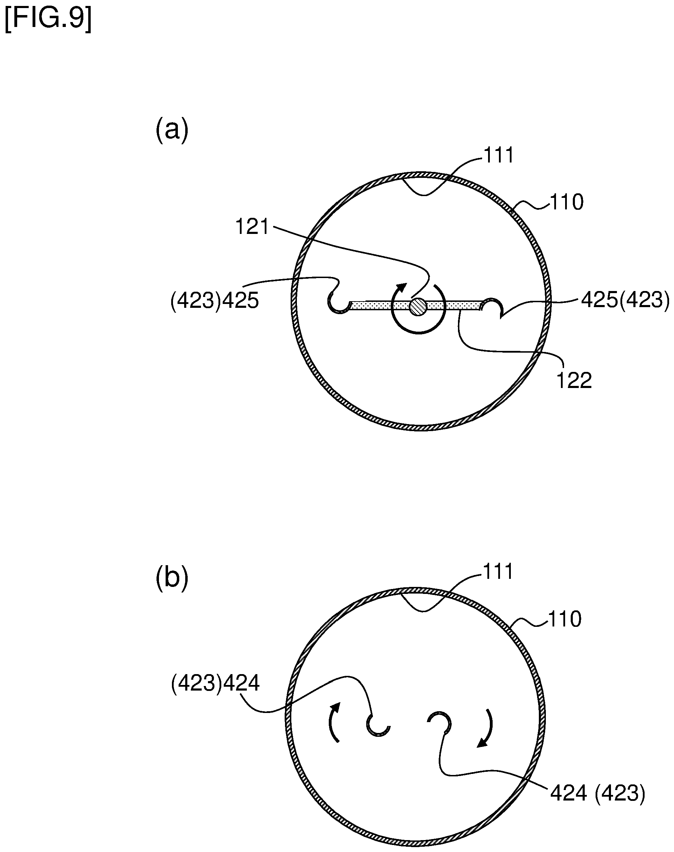

If such gutter-like liquid flow members 423 are employed, two liquid flow members 423 are attached around the axis of the rotary shaft 121 by means of the attachment 122 at a substantially equal distance from the rotary shaft 121 such that opening portions of the liquid flow members 423 are oriented in the moving direction (tangential direction) of the circular motion in a horizontal end face in the upper portion of the reaction chamber 110 in the reaction apparatus 400 (e.g., near ejection portions 425 of the liquid flow members 423 taken along the line A'-A' in FIG. 8) as shown in FIG. 9(a), for example. When the rotary shaft 121 rotates, the two liquid flow members 423 can be rotated via the attachment 122 along a portion closer to the inner wall 111 than to the center of the reaction chamber 110. Meanwhile, the two liquid flow members 423 are positioned close to the center of the reaction chamber 110 in a horizontal end face in the lower portion of the reaction chamber 110 in the reaction apparatus 400 (e.g., near lower ends 424 of the liquid flow members 423 taken along the line B'-B' in FIG. 8) as shown in FIG. 9(b), for example. When the rotary shaft 121 rotates, the two liquid flow members 423 can be rotated along a portion close to the center of the reaction chamber 110.

The rotation of the rotary shaft 121 can cause the gutter-like liquid flow members 423 as used in the reaction apparatus 400 in FIG. 8 to scoop up not only the reaction liquid 116 near the lower ends 425 (the water phase 116b in FIG. 8) but also the reaction liquid 116 positioned at portions in which the liquid members 223 is immersed above the lower ends 425 (the water phase 116b and the oil phase 116a in FIG. 8). The scooped up reaction liquid 116 is moved through the flow paths 426 of the flow liquid members 423 and then ejected from the ejection portions 425, and thus stirring or circulation of the reaction liquid 116 in the height direction of the reaction chamber 110 can be facilitated. The centrifugal force caused by the rotation of the rotary shaft 121 and applied to the lower portions of the liquid flow members 423 (e.g., the water phase 116b) is relatively small and the density of the reaction liquid 116 (the water phase 116b) that is scooped up is large, and thus the amount of liquid moving up through the flow paths 426 of the liquid flow members 423 may not be very much. Therefore, when such gutter-like liquid flow members 423 are used, the liquid flow members 423 can operate as conventional stirring blades as well.

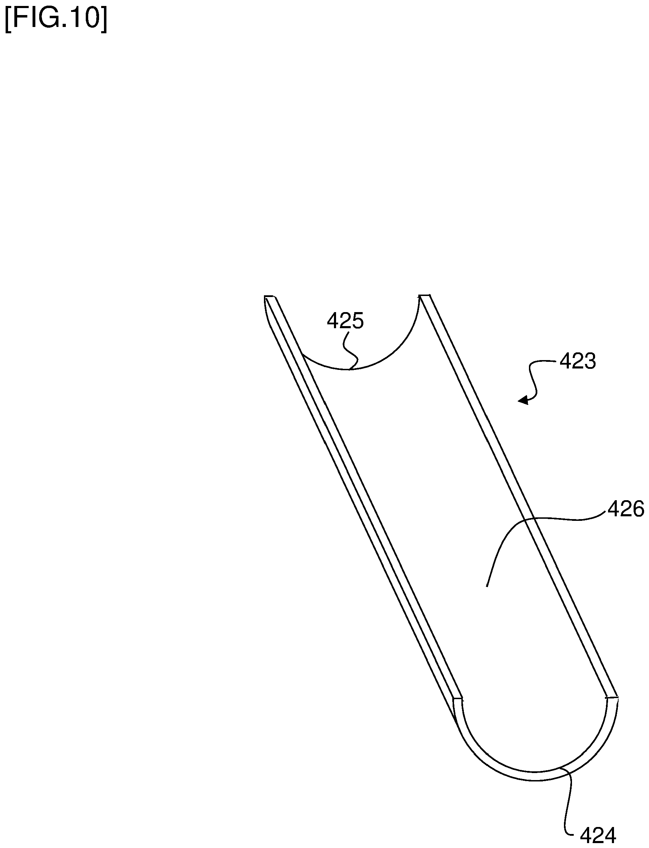

The size of the gutter-like liquid flow members 423 is not particularly limited either, but, for example, if a liquid flow member having a semi-circular cylindrical flow path 426 member as shown in FIG. 10 is employed, the inner diameter of the semi-circular cylindrical portion is 2 to 200 mm, for example. The length from the lower end 424 to the ejection portion 245 is 40 to 8,000 mm, for example. The width of the plate-like member is not necessarily limited, but is 20 to 300 mm, for example. The materials that can form the gutter-like liquid flow members 423 are the same as those mentioned above.

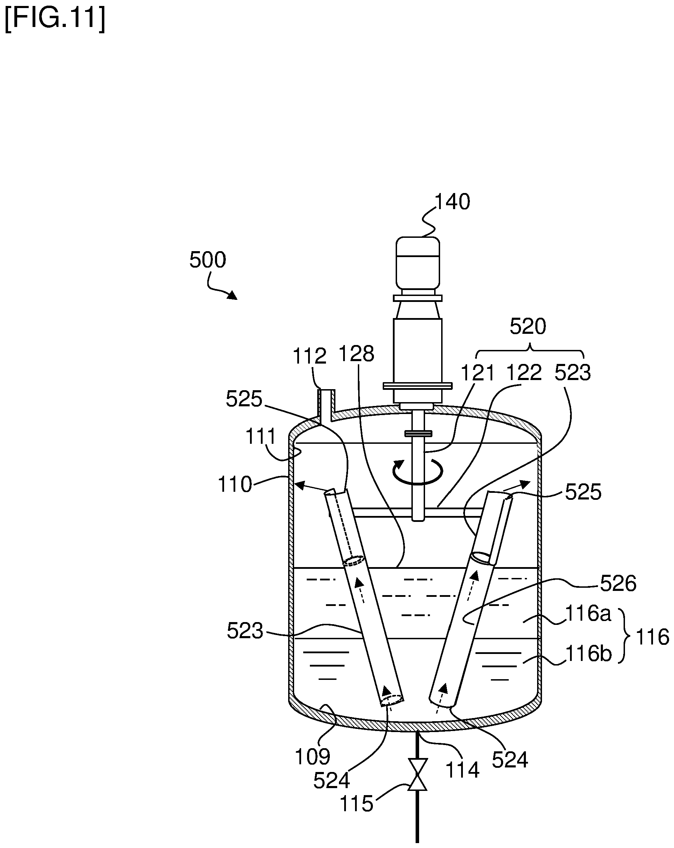

FIG. 11 is a schematic view showing another example of the reaction apparatus of the present invention. In a reaction apparatus 500 shown in FIG. 11, liquid flow members 523 constituting a liquid dispersion device 520 are each constituted by a combination of a cylindrical portion such as a circular cylindrical portion and a gutter-like portion. The liquid flow members 523 shown in FIG. 11 are designed such that their lower portions that are to be mainly immersed in the reaction liquid 116 in the reaction chamber 110 have a cylindrical shape and the other portions located above the lower portions have a gutter-like shape. In this arrangement, the reaction liquid 116 scooped up from suction portions 524 of the liquid flow members 523 is moved upward through flow paths 526 of the liquid flow members 523 due to the rotation of the rotary shaft 121, and then ejected from ejection portions 525 toward the inner wall 111 of the reaction chamber 110. The ejected reaction liquid can flow down as is along the inner wall 111 and be mixed with the reaction liquid 116 again.

In the embodiment shown in FIG. 11, the ratio of the cylindrical portion to the gutter-like portion constituting the liquid flow members 523 is not particularly limited. For example, an appropriate ratio may be selected by a person skilled in the art based on the capacity of the reaction chamber 110 and the amount of reaction liquid 116 contained therein (i.e., the amount of oil phase 116a and water phase 116b).

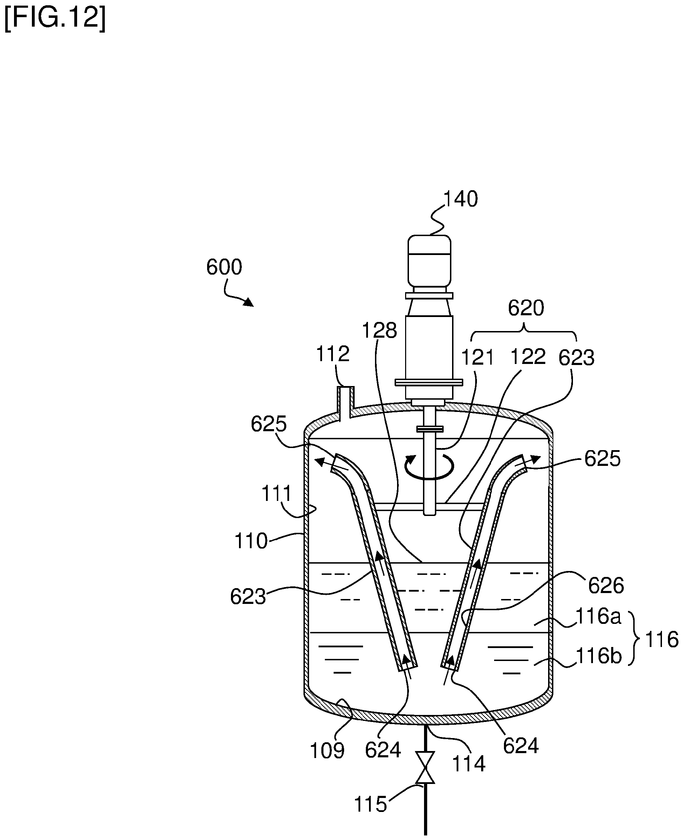

FIG. 12 is a schematic view showing another example of the reaction apparatus of the present invention. A reaction apparatus 600 shown in FIG. 12 is designed such that liquid flow members 623 constituting a liquid dispersion device 620 have cylindrical portions such as circular cylindrical portions and portions thereabove are curved to make ejection portions 625 oriented toward the inner wall 111 of the reaction chamber 110. In this arrangement, the reaction liquid 116 scooped up from suction portions 624 of the liquid flow members 623 is moved upward through flow paths 626 of the liquid flow members 623 due to the rotation of the rotary shaft 121, and then ejected from the ejection portions 625 toward the inner wall 111 of the reaction chamber 110. The ejected reaction liquid can flow down as is along the inner wall 111 and be mixed with the reaction liquid 116 again.

In the embodiment shown in FIG. 12, an example was described in which the liquid flow members 623 are gently curved at a predetermined curvature, for example, but the present invention is not limited to this. For example, it is also possible that the upper portion of each liquid flow member is bent at one or more points to make the ejection port oriented toward the inner wall 111 of the reaction chamber 110.

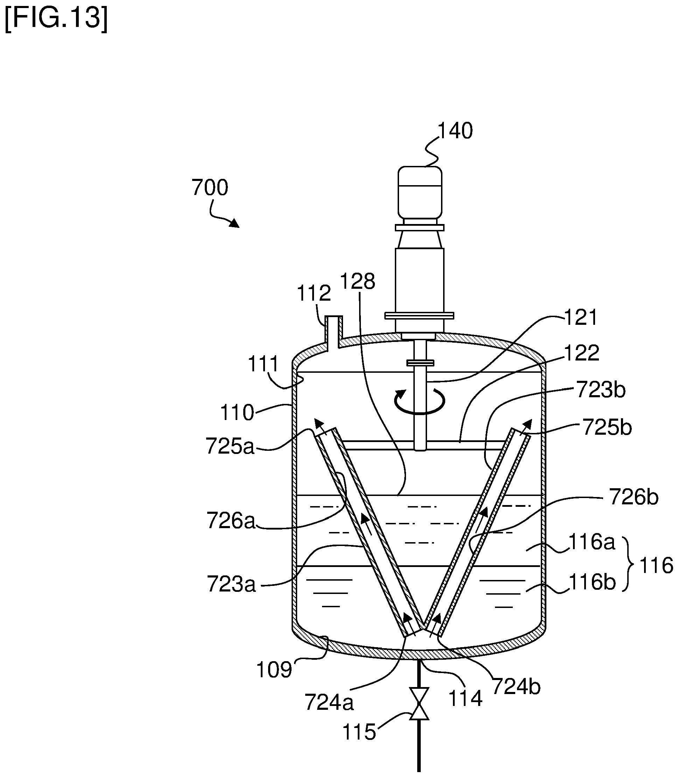

FIG. 13 is a schematic view showing another example of the reaction apparatus of the present invention. A reaction apparatus 700 shown in FIG. 13 is designed such that two circular cylindrical liquid flow members 723a and 723b constituting the liquid dispersion device face each other and their suction portions 724a and 724b are connected to each other. This arrangement allows the liquid flow members 723a and 723b to rotate more stably around the axis of the rotary shaft 121. The reaction liquid 116 scooped up from the suction portions 724a and 724b of the liquid flow members 723a and 723b is moved upward through flow paths 726a and 726b of the liquid flow members 723a and 723b due to the rotation of the rotary shaft 121, and then ejected from ejection portions 725a and 725b toward the inner wall 111 of the reaction chamber 110.

The reaction apparatus of the present invention is useful in the production of various reaction products where stirring of the reaction liquid (reactant) is desired. Such a reaction liquid may be constituted by one liquid (i.e., a single phase) or a plurality of liquid phases, for example. The reaction liquid may further contain predetermined particles (e.g., particles of immobilized enzymes or catalyst particles that are added to the reaction liquid) that are not dissolved but are precipitated or dispersed. Especially in a two-phase system (heterogeneous reaction system) such as those constituted by an oil phase and a water phase, the reaction product can be obtained more effectively than with a conventional stirrer. An example of such a two-phase system is a transesterification reaction to produce fatty acid esters.

### (Other Liquid Dispersion Devices)

Hereinafter, other examples of liquid dispersion devices that can be incorporated into this sort of reaction apparatus will be described.

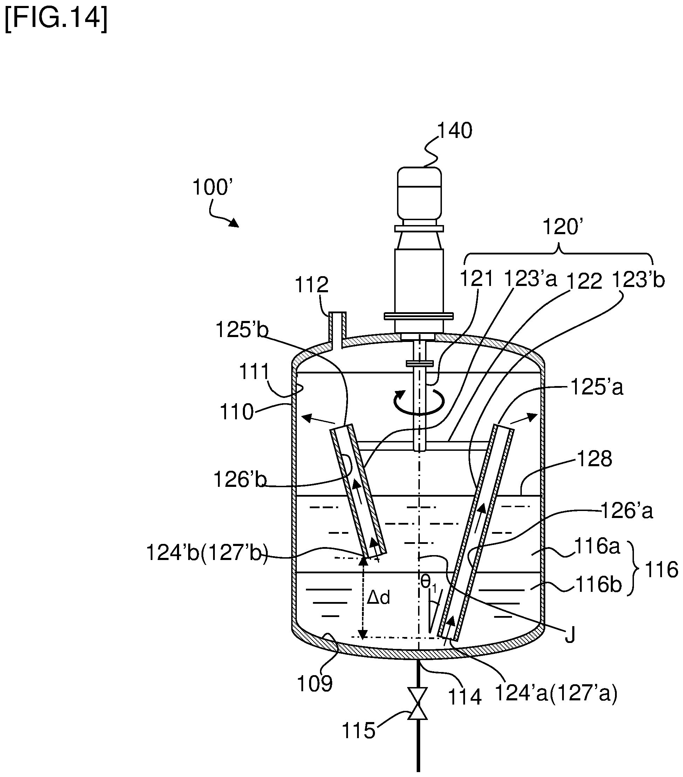

FIG. 14 is a schematic view showing an example of a reaction apparatus 100' incorporating another liquid dispersion device 120' of the present invention.

The liquid dispersion device 120' shown in FIG. 14 is constituted by the rotary shaft 121 disposed along the vertical direction, and a first liquid flow member 123'a and a second liquid flow member 123'b that are attached to the rotary shaft 121 via the attachment 122 extending preferably in the horizontal direction. The first liquid flow member 123'a includes a suction portion 124'a, an ejection portion 125'a, and a flow path 126'a extending between the suction portion 124'a and the ejection portion 125'a. The second liquid flow member 123'b includes a suction portion 124'b, an ejection portion 125'b, and a flow path 126'b extending between the suction portion 124'b and the ejection portion 125'b.

As shown in FIG. 14, in the liquid dispersion device 120', the suction portion 124'a of the first liquid flow member 123'a and the suction portion 124'b of the second liquid flow member 123'b are both arranged below the liquid surface 128 of the reaction liquid 116, and the ejection portion 125'a of the first liquid flow member 123'a and the ejection portion 125'b of the second liquid flow member 123'b are both arranged above the liquid surface 128 of the reaction liquid 116. Accordingly, the rotation of the rotary shaft 121 and centrifugal force caused by the rotation and applied to the first liquid flow member 123'a and the second liquid flow member 123'b can cause the liquid dispersion device 120' to scoop up the reaction liquid 116 contained in the reaction chamber 110 as shown in FIG. 14 from the suction portion 124'a of the first liquid flow member 123'a and the suction portion 124'b of the second liquid flow member 123'b, and cause the liquid to flow upward from the lower side of reaction chamber 110 through the flow paths 126'a and 126'b, for example. Subsequently, the scooped up reaction liquid is ejected from the ejection portion 125'a of the first liquid flow member 123'a and the ejection portion 125'b of the second liquid flow member 123'b to a position above the liquid surface 128.

The arrangement of the suction portions 124'a and 124'b and the ejection portions 125'a and 125'b relative to the liquid surface 128 is preferably maintained not only in a static stage (i.e., a state in which the rotary shaft 121 is not rotated and the liquid surface of the reaction liquid 116 is spread substantially in the horizontal direction) but also in a stage in which the rotary shaft 121 is rotated at a desired rotational speed (i.e., a state in which the reaction liquid 116 is stirred through the rotation of the rotary shaft 121). As a result, the reaction liquid 116 in the reaction chamber 110 can be easily scooped up from the suction portions 124'a and 124'b of the liquid flow members due to the rotation of the rotary shaft 121 and the liquid flow members 123'a and 123'b, moved by centrifugal force through the flow paths 126'a and 126'b in the liquid flow members 123'a and 123'b to the ejection portions 125'a and 125'b, and then ejected from the ejection portions 125'a and 125'b toward the inner wall 111 of the reaction chamber 110 and the liquid surface 128 of the reaction liquid 116, for example.

Furthermore, in the liquid dispersion device 120', the suction portion 124'a of the first liquid flow member 123'a is disposed above the suction portion 124'b of the second liquid flow member 123'b. That is to say, a lowermost end 127'a of the suction portion 124'a in the first liquid flow member 123'a and a lowermost end 127'b of the suction portion 124'b in the second liquid flow member 123'b are arranged at a distance Δd from each other in the vertical direction. The distance Δd is not necessarily limited because it varies in accordance with the sizes of the first liquid flow member 123'a and the second liquid flow member 123'b, the rotational speed applied to the rotary shaft 121, and the volume and depth of the reaction liquid 116 contained in the reaction chamber 110, but is, for example, 0.5 to 1,000 cm, and preferably 1 to 100 cm.

In an embodiment, if the reaction liquid 116 that is allowed to stand is constituted by a two-phase system including the oil phase 116a and the water phase 116b as shown in FIG. 14, the lowermost end 127'a of the suction portion 124'a in the first liquid flow member 123'a is positioned in the oil phase 116a that is allowed to stand, and the lowermost end 127'b of the suction portion 124'b in the second liquid flow member 123'b is positioned in the water phase 116b that is allowed to stand. Accordingly, the compositions of the reaction liquids 116 respectively scooped up by the suction portions 123'a and 123'b when the rotary shaft 121 rotates can be made different from each other rather than identical to each other.

In the embodiment shown in FIG. 14, a case was described in which the liquid dispersion device 120' including two liquid flow members (i.e., the first liquid flow member 123'a and the second liquid flow member 123'b) is used for the reaction liquid 116 constituted by a two-phase system including the oil phase 116a and the water phase 116b, but the present invention is not limited to this. For example, if the reaction liquid is constituted by a plurality of phases (e.g., three phases), a plurality of (e.g., three or more) liquid flow members whose lowermost ends are at different positions in the height direction may be provided for the purpose of scooping up the reaction liquid in respective phases.

In the liquid dispersion device 120' shown in FIG. 14, the first liquid flow member 123'a and the second liquid flow member 123'b are inclined such that the suction portions 124'a and 124'b are closer to an axis J of the rotary shaft 121 than the ejection portions 125'a and 125'b are. That is to say, in the liquid dispersion device 120', the first liquid flow member 123'a and the second liquid flow member 123'b are inclined such that the shortest distance between the suction portion 124'a of the first liquid flow member 123'a and the axis J of the rotary shaft 121 is shorter than that between the ejection portion 125'a and the axis J of the rotary shaft 121, and the shortest distance between the suction portion 124'b of the second liquid flow member 123'b and the axis J of the rotary shaft 121 is shorter than that between the ejection portion 125'b and the axis J of the rotary shaft 121.

Alternatively, in the liquid dispersion device 120' shown in FIG. 14, the first liquid flow member 123'a and the second liquid flow member 123'b are each attached so as to be inclined to form a predetermined angle (alternatively referred to as an "attachment inclination angle") θ₁ with respect to the axial direction of the rotary shaft 121. The attachment inclination angle θ₁ may be set to any angle by a person skilled in the art, but is, for example, 10 to 45°, and preferably 15 to 25°. The attachment inclination angles θ₁ of the first liquid flow member 123'a and the second liquid flow member 123'b may be the same or different from each other.

In the liquid dispersion device 120' shown in FIG. 14, the first liquid flow member 123'a and the second liquid flow member 123'b are provided facing each other via the axis J of the rotary shaft 121. The number of first liquid flow members that can be provided in the liquid dispersion device is not necessarily limited, but is, for example, at least one, preferably from 2 to 8, and more preferably from 2 to 6. The number of second liquid flow members that can be provided in the liquid dispersion device is not necessarily limited, but is, for example, at least one, preferably from 2 to 8, and more preferably from 2 to 6. If a plurality of first liquid flow members and a plurality of second liquid flow members are used, the first liquid flow members may be provided facing each other via the axis of the rotary shaft and the second liquid flow members may be provided facing each other via the axis of the rotary shaft. Furthermore, the first and second liquid flow members are preferably attached at substantially equal angle intervals around the axis of the rotary shaft.

The first liquid flow member 123'a and the second liquid flow member 123'b may be processed to be entirely cylindrical (e.g., circular cylindrical, elliptic cylindrical, or angular cylindrical), may have a so-called gutter-like shape such as a semi-circular cylindrical, semi-angular cylindrical, or V letter shape at the lower end and the upper end with an intermediate portion therebetween being processed to be cylindrical (e.g., circular cylindrical, elliptic cylindrical, or angular cylindrical), or may have such a gutter-like shape only at the lower end with the other portion being processed to be cylindrical (e.g., circular cylindrical, elliptic cylindrical, or angular cylindrical), for example.

The sizes of the first liquid flow member 123'a and the second liquid flow member 123'b are not particularly limited. For example, in the case of the second liquid flow member 123'b, if a circular cylindrical member is used in the second liquid flow member 123'b, the inner diameter of the circular cylindrical portion is 2 to 200 mm, for example. The length from the suction portion 124'b to the ejection portion 125'b (i.e., the length of the path 126'b) is not necessarily limited, and an appropriate length may be selected by a person skilled in the art according to the length selected for the first liquid flow member 123'a, the size of the reaction chamber into which the liquid flow members are incorporated, the size of the attachment inclination angle θ₁, and the like, for example. In FIG. 14, the inner diameter of the suction portion 124b, the inner diameter of the path 126'b, and the inner diameter of the ejection portion 125'b are shown as if they were of the same size, but the present invention is not limited to this. For example, the inner diameter of the second liquid flow member 123'b may gradually or stepwise decrease from the suction portion 124'b to the ejection portion 125'b, or the inner diameter of the second liquid flow member 123'b may gradually or stepwise increase from the suction portion 124'b to the ejection portion 125'b.

The liquid dispersion device 120' can also be incorporated into a mixing or stirring chamber of a mixing or stirring device used when physical mixing or stirring of the contents is required, for example, in addition to the reaction chamber 110 of the reaction apparatus 100' as shown in FIG. 14.

### (Method for Producing Reaction Product)

Next, a method for producing a predetermined reaction product using the reaction apparatus of the present invention will be described.

In the production method of the present invention, stirring is performed through circulation of the reaction liquid in the above-described reaction apparatus. Here, the term "stirring through circulation" as used herein refers to both stirring by applying horizontal rotation to a target liquid (e.g., a reaction liquid) and mixing up (or mixing) of the entire liquid through vertical movement and circulation of the liquid, as in the case of using the above-described reaction apparatus.

The reaction liquid for use in the present invention contains an inorganic or organic liquid medium, and its chemical reaction is generally progressed and controlled through stirring with a stirrer or the like. For example, a heterogeneous reaction liquid is used as the reaction liquid. For example, a reaction liquid constituted by an oil phase and a water phase is useful in that the emulsification of the reaction liquid can be improved and facilitated by the above-mentioned stirring through circulation.

If the reaction liquid is a heterogeneous reaction liquid, the reaction liquid contains a raw material fat or oil, a liquid enzyme, an alcohol having 1 to 8 carbon atoms, and water, for example.

The raw material fat or oil is, for example, a fat or oil that can be used to produce a fatty acid ester for biodiesel fuel. The raw material fat or oil may be a preliminarily refined fat or oil or may be an unrefined fat or oil containing impurities. Examples of the raw material fat or oil include cooking fats and oils and waste cooking fats and oils thereof, crude oils, and other waste matter-based fats and oils, as well as combinations thereof. Examples of the cooking fats and oils and waste cooking fats and oils thereof include vegetable fats and oils, animal fats and oils, fish oils, fats and oils produced by microorganisms, and waste oils thereof, as well as mixtures (mixed fats and oils) thereof. Examples of the vegetable fats and oils include, but not necessarily limited to, soybean oil, rapeseed oil, palm oil, and olive oil. Examples of the animal fats and oils include, but not necessarily limited to, beef tallow, lard, chicken fat, whale oil, and mutton tallow. Examples of the fish oils include, but not necessarily limited to, sardine oil, tuna oil, and squid oil. Examples of the fats and oils produced by microorganisms include, but not necessarily limited to, fats and oils produced by microorganisms belonging to the genus *Mortierella,* the genus *Schizochytrium,* or the like.

Examples of the crude oils include unrefined or unprocessed fats and oils that are obtained from a conventional oil expression step for cooking fats and oils, and the crude oils may contain, for example, gum-like impurities, such as phospholipids and/or proteins, free fatty acids, pigments, trace metals, and other hydrocarbon impurities that are soluble in oil, as well as combinations thereof. The amount of impurities contained in a crude oil is not limited.

Examples of the other waste matter-based fats and oils include oil foots obtained as a result of refining, in the presence of an alkali, raw oil that is produced during the production of a food fat or oil, heat-treatment oil, press oil, and rolling oil, as well as combinations thereof.

The raw material fat or oil may contain water in any amount, as long as the water does not inhibit the inherent characteristics of the fat or oil. Furthermore, an unreacted fat or oil remaining in a solution that has been used in a separate reaction for forming a fatty acid ester may also be used as the raw material fat or oil.

With regard to the liquid enzyme in the present invention, enzyme catalysts that have the properties of liquid at room temperature, of any enzyme catalysts that can be used for a fatty acid ester generating reaction can be used. Examples of the liquid enzyme include lipase, cutinase, and combinations thereof. Here, the term "lipase" as used herein refers to an enzyme that has the ability to act on a glyceride (also called acylglycerol) and degrade the glyceride into glycerin or a partial glyceride and a fatty acid, and also has the ability to generate a fatty acid ester via transesterification in the presence of a linear lower alcohol.

The lipase in the present invention may be 1,3- specific or may be nonspecific. In terms of the capability of producing a linear lower alcohol ester of a fatty acid, it is preferable that the lipase is nonspecific. Examples of the lipase include lipases derived from filamentous fungi belonging to the genus *Rhizomucor* (*Rhizomucor miehei*)*,* the genus *Mucor,* the genus *Aspergillus,* the genus *Rhizopus,* the genus *Perzicillium,* and the like; lipases derived from yeasts belonging to genus *Candida* (*Candida antarcitica, Candida rugosa,* and *Candida cylindracea*)*, Pichia,* and the like; lipases derived from bacteria belonging to the genus *Pseudomonas,* the genus *Serratia,* and the like; and lipases derived from animals, such as hog pancreas. Liquid lipase can be obtained by, for example, concentrating and refining a culture solution of any of the above-described microorganisms containing lipase produced by that microorganism or by dissolving powdered lipase in water. A commercially available liquid lipase can also be used.

The amount of the above-described liquid enzyme used in the present invention varies in accordance with, for example, the type and/or amount of raw material fat or oil and is therefore not necessarily limited, but may be preferably 0.1 to 50 parts by mass, and more preferably 0.2 to 30 parts by mass, with respect to 100 parts by mass of raw material fat or oil that is used. When the amount of liquid enzyme that is used is less than 0.1 parts by mass, an effective transesterification reaction cannot be catalyzed, and there is thus a risk that the yield and/or the percentage yield of a desired fatty acid ester may be reduced. When the amount of liquid enzyme that is used exceeds 50 parts by mass, the yield and/or the percentage yield of the desired fatty acid ester that is obtained through the transesterification reaction no longer changes, but rather there is a risk that the production efficiency may be reduced.

The alcohol that is used in the present invention is a linear or branched lower alcohol (e.g., an alcohol having 1 to 8 carbon atoms, and preferably an alcohol having 1 to 4 carbon atoms). A linear lower alcohol is preferable. Examples of the linear lower alcohol include, but not necessarily limited to, methanol, ethanol, n-propanol, and n-butanol, as well as combinations thereof.

The amount of the above-described alcohol varies in accordance with, for example, the type and/or amount of raw material fat or oil that is used and is therefore not necessarily limited, but may be preferably 5 to 100 parts by mass, and more preferably 10 to 30 parts by mass, with respect to 100 parts by mass of raw material fat or oil. When the amount of alcohol that is used is less than 5 parts by mass, an effective transesterification reaction cannot be realized, and there is thus a risk that the yield and/or the percentage yield of a desired fatty acid ester may be reduced. When the amount of alcohol that is used exceeds 100 parts by mass, the yield and/or the percentage yield of the desired fatty acid ester that is obtained through the transesterification reaction no longer changes, but rather there is a risk that the production efficiency may be reduced.

Water that is used in the present invention may be any of distilled water, ion-exchanged water, tap water, and pure water. The amount of the above-described water used varies in accordance with, for example, the type and/or amount of raw material fat or oil that is used, and is therefore not necessarily limited, but may be preferably 0.1 to 50 parts by mass, and more preferably 2 to 30 parts by mass, with respect to 100 parts by mass of raw material fat or oil. When the amount of water that is used is less than 0.1 parts by mass, the amount of the water layer formed in the reaction system is insufficient, making it impossible for an effective transesterification reaction using the above-described raw material fat or oil, liquid enzyme, and alcohol to occur, and there is thus a risk that the yield and/or the percentage yield of a desired fatty acid ester may be reduced. When the amount of water that is used exceeds 50 parts by mass, the yield and/or the percentage yield of the desired fatty acid ester that is obtained through the transesterification reaction no longer changes, but rather there is a risk that the production efficiency may be reduced.

In the production method of the present invention, a predetermined electrolyte may be added to the above-described reaction liquid. Examples of anions constituting the electrolyte include, but not necessarily limited to, hydrogen carbonate ions, carbonate ions, chloride ions, hydroxide ions, citrate ions, hydrogen phosphate ions, dihydrogen phosphate ions, and phosphate ions, as well as combinations thereof. Examples of cations constituting the electrolyte include alkali metal ions and alkaline earth metal ions as well as combinations thereof, and more specifically include sodium ions, potassium ions, and calcium ions as well as combinations thereof. In the present invention, preferred examples of the electrolyte include sodium hydrogen carbonate (baking soda), sodium carbonate, calcium chloride, calcium hydroxide, trisodium citrate, sodium hydrogen phosphate, sodium dihydrogen phosphate, sodium chloride, and trisodium phosphate, as well as combinations thereof. Sodium hydrogen carbonate (baking soda) is more preferable because it is versatile and easily available, for example.

In the present invention, the above-described raw material fat or oil, catalyst, alcohol, and water are placed into the reaction chamber 110 of the reaction apparatus 100 shown in FIG. 1 through the reaction liquid inlet 112 simultaneously or in any order to form a reaction liquid 116 constituted by an oil phase 116a and a water phase 116b, for example. Subsequently, when the liquid flow members 123 of the liquid dispersion device 120 are rotated inside the reaction chamber 110 through the rotation of the rotary shaft 121, as described above, the reaction liquid 116 is scooped up from the suction portions 124 of the liquid flow members 123, and the scooped up reaction liquid is moved upward through the flow paths 126 and then ejected from the ejection ports 125 of the liquid flow members 123. This movement of the reaction liquid 116 facilitates the stirring of the reaction liquid 116 and the formation of a fatty acid ester that is a reaction product. The temperature applied in the reaction chamber 110 is not necessarily limited, but is, for example, 5 to 80°C, preferably 15 to 80°C, and more preferably 25 to 50°C.

It should be noted that, in the present invention, the rotation of the rotary shaft inside the reaction apparatus 100 is not necessarily required to be performed at a high speed (e.g., 600 rpm or higher). For example, the rotation may be set to a low speed (e.g., 80 rpm or higher and lower than 300 rpm) or a middle speed (e.g., 300 rpm or higher and lower than 600 rpm). Furthermore, the reaction time varies in accordance with the amount of each of the raw material fat or oil, catalyst, alcohol, and water, and is therefore not necessarily limited. Any desired period of time may be set by a person skilled in the art.

After the completion of the reaction, the product and reaction residues are taken out of the reaction chamber 110 of the reaction apparatus 100, and separated into a layer containing the fatty acid ester and a layer containing the by-product glycerin by using, for example, a method that is well-known to a person skilled in the art. After that, the layer containing the fatty acid ester may be further subjected to isolation and refining of the fatty acid ester by using a method that is well-known to a person skilled in the art, as necessary.

The fatty acid ester that is obtained in the above-described manner can be used as, for example, a biodiesel fuel or a constituent component thereof.

### Examples

Hereinafter, the present invention will be described in detail using examples. However, the present invention is not limited to these examples.

### (Reference Example 1: Fabrication of Test Apparatus (R1))

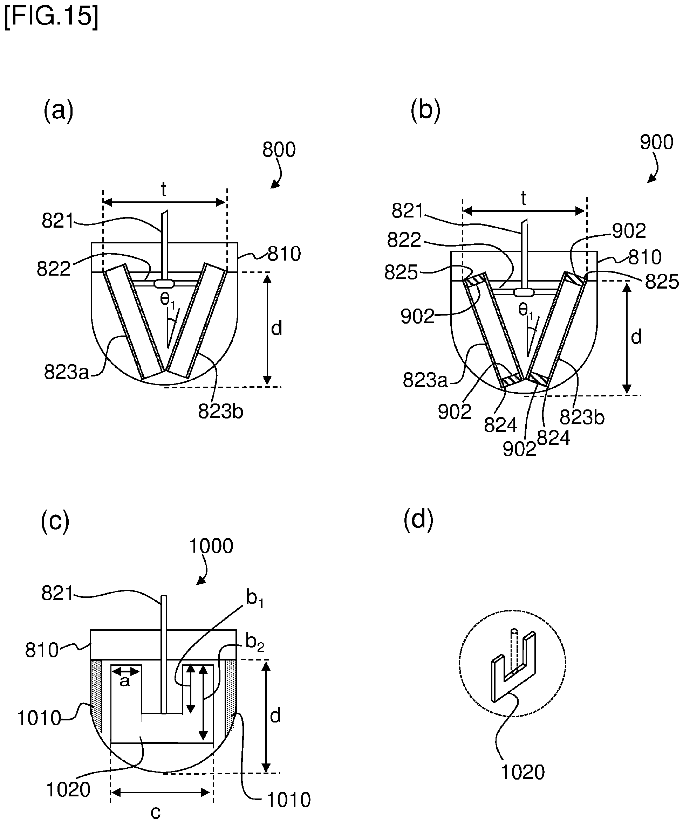

A test apparatus (R1) 800 shown in FIG. 15(a) was fabricated as follows. Specifically, two circular cylindrical stainless steel pipes 823a and 823b (with an inner diameter of 10 mm and a length of 86 mm) were fixed in a V-shape form with an attachment 822 so as to be arranged facing each other and inclined at 20° with respect to the axial direction of a rotary shaft 821 in a round-bottomed and transparent reaction chamber 810 with an inner diameter of 115 mm. A distance t between the upper ends of the pipes 823a and 823b was 84 mm.

### (Reference Example 2: Fabrication of Test Apparatus (R2))

A test apparatus (R2) 900 shown in FIG. 15(b) was fabricated as follows. Specifically, the test apparatus (R2) 900 was fabricated in the same way to that of Reference Example 1, except that lower ends 824 and upper ends 825 of the pipes 823a and 823b constituting the test apparatus (R1) 800 shown in FIG. 15(a) were sealed with silicone resin 902.

### (Reference Example 3: Fabrication of Test Apparatus (R3))

A test apparatus (R3) 1000 shown in FIG. 15(c) was fabricated as follows. Specifically, the test apparatus (R3) 1000 was fabricated by arranging an anchor-type (U-shaped) stirring blade 1020 shown in FIG. 15(d) instead of the pipes 823a and 823b constituting the test apparatus (R1) 800 shown in FIG. 15(a) and arranging three 11.5 mm wide obstacle plates 1010 (only two are shown in FIG. 15(c)) at substantially equal intervals in the horizontal direction on the inner wall of reaction chamber 810. The size of the U-shaped stirring blade was set such that *a* was 15 mm, *b₁* was 35 mm, *b₂* was 50 mm, and *c* was 65 mm.

### (Reference Example 4: Relationship between Number of Rotations and Torque of Test Apparatus)

The relationship between the number of rotations and the torque in the test apparatus (R1) fabricated in Reference Example 1 and the test apparatus (R3) fabricated in Reference Example 3 was measured as follows.

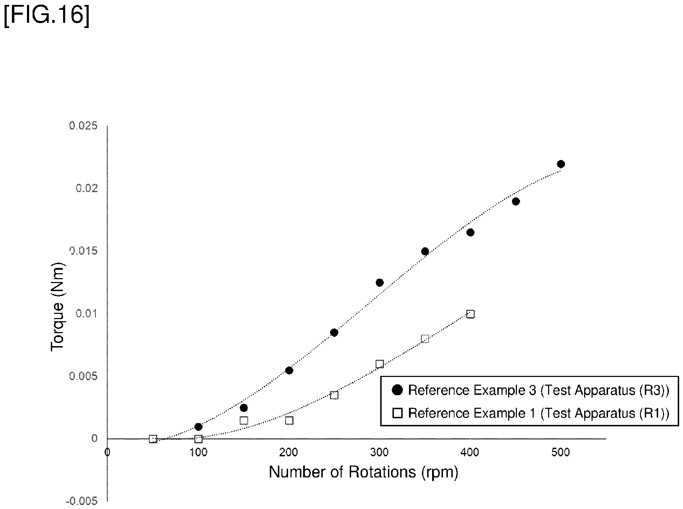

Specifically, methyl ester was placed into the reaction chamber 810 of each of the test apparatus (R1) fabricated in Reference Example 1 shown in FIG. 15(a) and the test apparatus (R3) fabricated in Reference Example 3 shown in FIG. 15(c) such that a height d from the bottom to the liquid surface was 85 mm. In this state, the number of rotations of an unshown motor was increased from 0 to 500 rpm, and a torque (N·m) was measured using a torque transducer-equipped stirrer BL1200Te (manufactured by Shinto Scientific Co., Ltd.). FIG. 16 shows the obtained results.

As shown in FIG. 16, it is seen that the test apparatus (R1) fabricated in Reference Example 1 can rotate the circular cylindrical pipes (823a and 823b shown in FIG. 15(a)) with significantly lower torque and less power at the same number of rotations compared with the test apparatus (R3) of Reference Example 3.

### (Example 1: Production of Methyl Esters using Test Apparatus (R1)

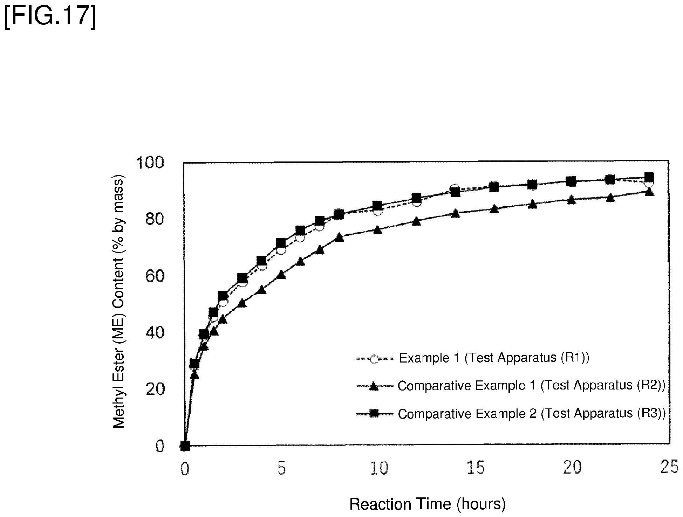

In this example, 500 g of palm oil with an acid value of 0.423 mg-KOH/g, 10 g of liquid enzyme (liquid lipase; Callera Trans L, manufactured by Novozymes), 75 g of distilled water, and 5 M equivalents of methanol were placed into the reaction chamber 810 of the test apparatus (R1) 800 obtained in Reference Example 1 shown in FIG. 15(a), and a transesterification reaction was caused to occur while keeping the internal portion of the reaction chamber 810 at 40°C and setting the rotational speed of the rotary shaft 821 to 200 rpm. During the reaction, the reaction liquid in the reaction chamber 810 was periodically sampled, and the methyl ester (ME) content in the reaction liquid was measured using gas chromatography (GC-2010 manufactured by Shimadzu Corporation). FIG. 17 shows the obtained results.

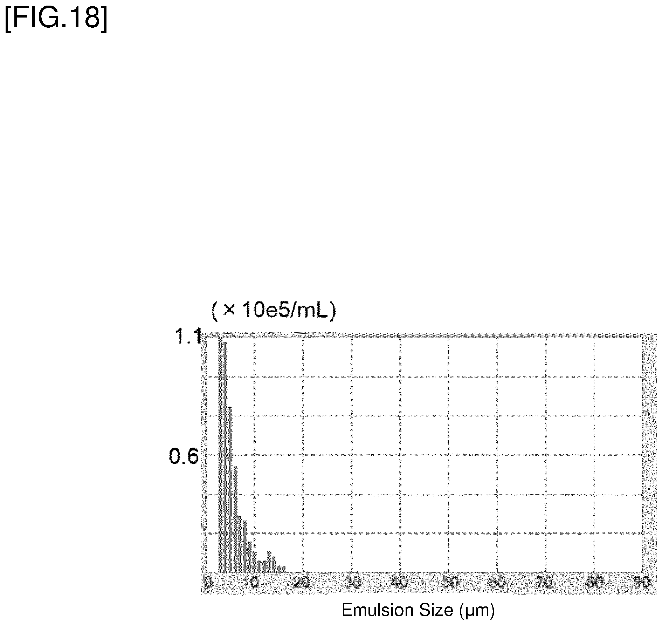

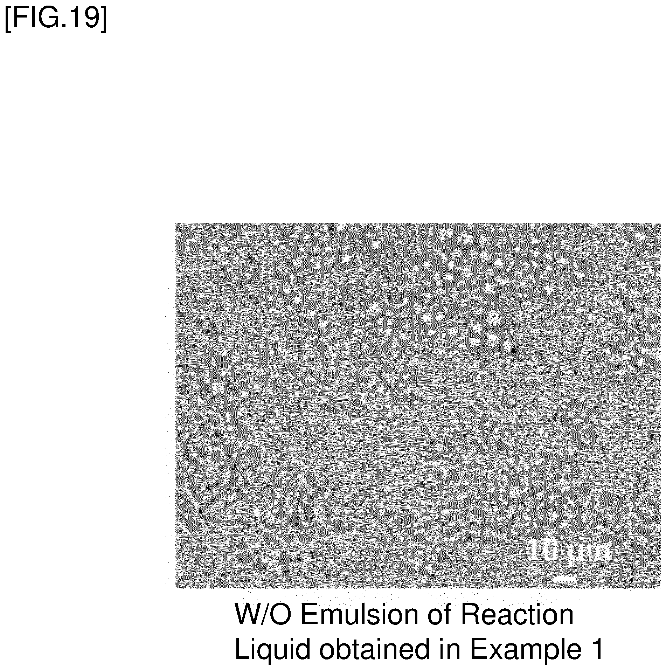

Then, 24 hours after starting the transesterification reaction, the reaction liquid in the reaction chamber 810 was sampled, and the particle size distribution of the W/O emulsion of the obtained reaction liquid was measured using an automatic cell counter (Model R1 manufactured by Olympus Corporation). FIG. 18 shows the obtained results. Also, the W/O emulsion of this reaction liquid was observed with a biological microscope (CX21LED manufactured by Olympus Corporation) at a magnification of 1,000x (FIG. 19).

### (Comparative Example 1: Production of Methyl Esters using Test Apparatus (R2))

A transesterification reaction was caused to occur in the same way to that of Example 1 while keeping the rotational speed of the rotary shaft 821 at 200 rpm, except that the test apparatus (R2) 900 obtained in Reference Example 2 shown in FIG. 15(b) was used instead of the test apparatus (R1) used in Example 1. During the reaction, the reaction liquid in the reaction chamber 810 was periodically sampled, and the methyl ester (ME) content in the reaction liquid was measured using gas chromatography in the same way to that of Example 1. FIG. 17 shows the obtained results.

### (Comparative Example 2: Production of Methyl Esters using Test Apparatus (R3))

A transesterification reaction was caused to occur in the same way to that of Example 1 while keeping the rotational speed of the rotary shaft 821 at 200 rpm, except that the test apparatus (R3) 1000 obtained in Reference Example 3 shown in FIG. 15(c) was used instead of the test apparatus (R1) used in Example 1. During the reaction, the reaction liquid in the reaction chamber 810 was periodically sampled, and the methyl ester (ME) content in the reaction liquid was measured using gas chromatography in the same way to that of Example 1. FIG. 17 shows the obtained results.

As shown in FIG. 17, the reaction system of Example 1 using the test apparatus (R1) with both ends of each of the circular cylindrical pipes 823a and 823b open clearly produced a larger amount of more methyl ester (ME) immediately after the reaction started, compared with the reaction system of Comparative Example 1 using the test apparatus (R2) with both ends of each of the circular cylindrical pipes 823a and 823b sealed with the silicone resin 902. In addition, when the circular cylindrical pipes 823a and 823b of the test apparatuss (R1) and (R2) were checked during the actual reaction, it was seen that the reaction liquid was ejected from the upper ends of the circular cylindrical pipes 823a and 823b in the test apparatus (R1) as the rotary shaft 821 rotated, which indicates that the reaction liquid was more effectively circulated and stirred compared with that in the test apparatus (R2). This indicates that, in the reaction system of Example 1 using the test apparatus (R1), the reaction efficiency of methyl ester (ME) was enhanced by the circular cylindrical pipes 823a and 823b with open ends.

On the other hand, as shown in FIG. 17, comparing the reaction system of Example 1 using the test apparatus (R1) and the reaction system of Comparative Example 2 using the test apparatus (R3) with the anchor-type stirring blade, there was no significant difference in the amount of methyl ester (ME) produced from immediately after the reaction started. In both the reaction system of Example 1 and the reaction system of Comparative Example 2, the rotational speed of the rotary shaft 821 was 200 rpm. Here, as shown in FIG. 16, as for the torque at the same rotational speed (e.g., 200 rpm) of the test apparatus (R1) of Reference Example 1 and the test apparatus (R3) of Reference Example 3, the torque of the test apparatus (R3) of Reference Example 3 was clearly higher than that of the test apparatus (R1) of Reference Example 1, which indicates that more power is required. Accordingly, it is seen that the reaction system of Example 1 using the test apparatus (R1) produced methyl ester (ME) with less power compared with the reaction system of Comparative Example 2 using the test apparatus (R3) that required more power.

Furthermore, as shown in FIGS. 18 and 19, in the reaction system of Example 1 using the test apparatus (R1), a W/O emulsion of the reaction liquid was formed with a relatively narrow particle size distribution and a suitable size for the transesterification reaction. Furthermore, it was seen from the graph shown in FIG. 18 that the obtained W/O emulsion had an average particle size of 5.9 µm.

### (Example 2: Production of Methyl Esters using Test Apparatus (R1))

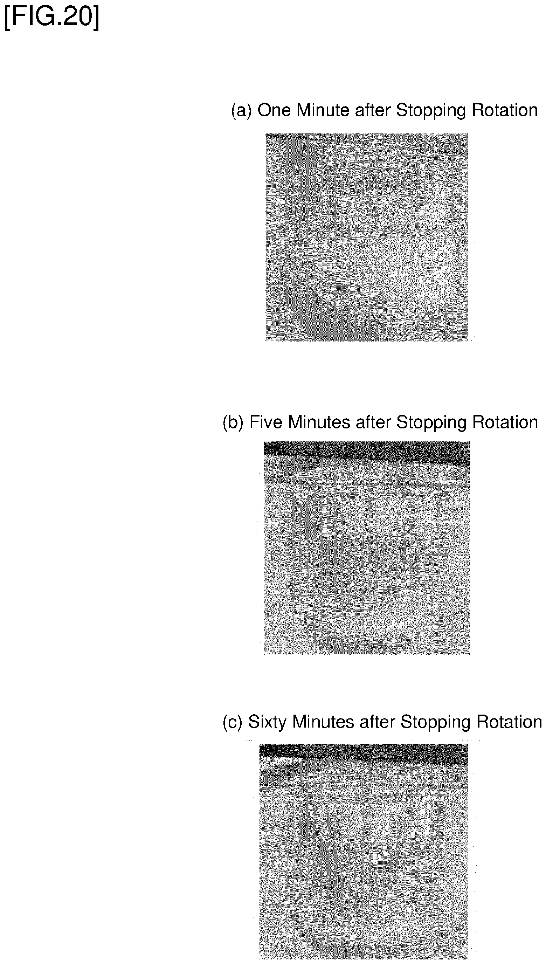

A transesterification reaction was caused to occur in the same way to that of Example 1, except that the rotational speed of the rotary shaft 821 was changed to 300 rpm. Then, 24 hours after starting the reaction, the rotation of the rotary shaft 821 was stopped, and photographs were taken of the state of the reaction liquid in the reaction chamber 810 after 1 minute, 5 minutes, and 60 minutes had elapsed. FIG. 20 shows the obtained results.

It was seen from FIG. 20 that, when 60 minutes (FIG. 20(c)) had elapsed after the rotary shaft 821 was stopped, the W/O emulsion was lost and the oil and water phases were almost phase separated in the reaction chamber 810, compared with when 1 minute had elapsed (FIG. 20(a)) and when 5 minutes had elapsed (FIG. 20(b)).

### Industrial Applicability

According to the present invention, products such as a fatty acid ester can be efficiently produced. The fatty acid ester that is obtained according to the present invention is useful as, for example, a biodiesel fuel or a constituent component thereof.

### Reference Signs List

- 100, 100', 200, 200a, 300, 400, 500, 600, 700: Reaction apparatus
- 109: Bottom
- 110, 810: Reaction chamber
- 111: Inner wall
- 112: Reaction liquid inlet
- 114: Product outlet
- 115: Valve
- 116: Reaction liquid
- 116a: Oil phase
- 116b: Water phase
- 120, 120', 420: Liquid dispersion device
- 121, 721: Rotary shaft
- 122,722: Attachment
- 123, 123'a, 123'b, 423, 523, 623, 723a, 723b: Liquid flow member
- 124, 124'a, 124'b, 624, 724: Suction portion
- 125, 125'a, 125'b, 425, 525, 625, 725a, 725b: Ejection portion
- 126, 126'a, 126'b, 426, 526, 626, 726a, 726b: Flow path
- 128: Liquid surface
- 130: Jacket
- 131: Jacket inlet
- 137: Jacket outlet
- 140: Motor
- 210, 1010: Obstruction plate
- 220: Inverted skirt
- 222: Upper opening
- 224: Lower opening
- 226: Inner inclined face
- 228: Outer inclined face
- 800, 900, 1000: Test apparatus
- 823a, 823b: Pipe
- 902: Silicone resin
- 1020: Anchor-type stirring blade

## Claims

1. A reaction apparatus comprising: a reaction chamber that contains a reaction liquid; and a liquid dispersion device provided inside the reaction chamber,
wherein the liquid dispersion device includes a rotary shaft disposed along a vertical direction and at least one liquid flow member attached to the rotary shaft, and
the liquid flow member includes an ejection portion positioned above a liquid surface of the reaction liquid, a suction portion positioned below the liquid surface of the reaction liquid, and a flow path extending between the ejection portion and the suction portion and allowing the reaction liquid to flow therethrough.

2. The reaction apparatus of claim 1, wherein the liquid flow member is inclined such that the suction portion is closer to the rotary shaft than the ejection portion is.

3. The reaction apparatus of claim 1 or 2, wherein the liquid dispersion device includes a plurality of the liquid flow members around an axis of the rotary shaft.

4. The reaction apparatus of any one of claims 1 to 3, wherein the liquid flow member has a cylindrical shape with open ends.

5. The reaction apparatus of any one of claims 1 to 3, wherein the liquid flow member has a gutter-like shape.

6. The reaction apparatus of any one of claims 1 to 5, wherein the liquid flow member is constituted by at least one first liquid flow member and at least one second liquid flow member, and the suction portion of the first liquid flow member is disposed above the suction portion of the second liquid flow member.

7. The reaction apparatus of any one of claims 1 to 6, wherein the reaction liquid is constituted by a plurality of liquid phases.

8. A liquid dispersion device comprising: a rotary shaft disposed along a vertical direction; and at least one first liquid flow member and at least one second liquid flow member that are attached to the rotary shaft,
wherein the first liquid flow member and the second liquid flow member each include an ejection portion positioned at an upper end thereof, a suction portion positioned at a lower end, and a flow path extending between the ejection portion and the suction portion, and
the suction portion of the first liquid flow member is disposed above the suction portion of the second liquid flow member.

9. The liquid dispersion device of claim 8, wherein the first liquid flow member and the second liquid flow member are each inclined such that the suction portion is closer to an axis of the rotary shaft than the ejection portion is.

10. The liquid dispersion device of claim 8 or 9, wherein the first liquid flow member and the second liquid flow member each have a cylindrical shape with open ends.

11. A method for producing a reaction product, comprising stirring a reaction liquid through circulation inside the reaction apparatus of any one of claims 1 to 7.

12. The method of claim 11, wherein the reaction liquid is constituted by a plurality of liquid phases.

13. The method of claim 11 or 12, wherein the reaction product is a fatty acid ester, and the reaction liquid contains a raw material fat or oil, a liquid enzyme, an alcohol having 1 to 8 carbon atoms, and water.

14. The method of claim 13, wherein the raw material fat or oil is at least one fat or oil selected from the group consisting of vegetable fats and oils, animal fats and oils, fish oils, fats and oils produced by microorganisms, and waste oils thereof.
